# EUROPEAN PATENT SPECIFICATION

(11) **EP 1 761 642 B1**
(45) Date of publication and mention of the grant of the patent: **12.11.2008**
(21) Application number: 05741102.7
(22) Date of filing: 02.05.2005
(51) Int. Cl.: C12Q 1/68

(54) **Use of the genes Kir2.3 and DRD2 as molecular markers for the diagnosis of schizophrenia**
Verwendung der Gene Kir2.3 und DRD2 als molekulare Marker zur Diagnose von Schizophrenie
Utilisation des gênes Kir2.3 et DRD2 comme marqueurs moléculaires dans le diagnostic de la schizophrénie

(30) Priority: 30.04.2004 HU 0400904 P
(43) Date of publication of application: 14.03.2007
(73) Proprietor: The Biological Research Center of the Hungarian Academy of Sciences, 6701 Szeged (HU)
(72) Inventor: PUSKÁS, László, H-6753 Szeged (HU); ZVARA, Ágnes, H-6725 Szeged (HU); SÁNTHA, Miklós, H-6725 Szeged (HU); SZEKERES, György, H-6726 Szeged (HU); JANKA, Zoltán, H-6720 Szeged (HU)
(74) Representative: Svingor, Adam
(86) International application number: PCT/HU2005/000044
(87) International publication number: WO 2005/106026

(56) References cited:
- EP-A- 1 132 483
- EP-A- 1 348 963
- WO-A-02/14547
- TALLERICO T ET AL: "Schizophrenia: elevated mRNA for dopamine D2(Longer) receptors in frontal cortex." BRAIN RESEARCH. MOLECULAR BRAIN RESEARCH. 5 MAR 2001, vol. 87, no. 2, 5 March 2001 (2001-03-05), pages 160-165, XP002343957 ISSN: 0169-328X
- ABI-DARGHAM A ET AL: "Increased baseline occupancy of D2 receptors by dopamine in schizophrenia." PROCEEDINGS OF THE NATIONAL ACADEMY OF SCIENCES OF THE UNITED STATES OF AMERICA. 5 JUL 2000, vol. 97, no. 14, 5 July 2000 (2000-07-05), pages 8104-8109, XP002343958 ISSN: 0027-8424
- SEEMAN P ET AL: "Schizophrenia: normal sequence in the dopamine D2 receptor region that couples to G-proteins. DNA polymorphisms in D2." NEUROPSYCHOPHARMACOLOGY : OFFICIAL PUBLICATION OF THE AMERICAN COLLEGE OF NEUROPSYCHOPHARMACOLOGY. FEB 1993, vol. 8, no. 2, February 1993 (1993-02), pages 137-142, XP008052203 ISSN: 0893-133X
- BUDARF M L ET AL: "Assignment of the human hippocampal inward rectifier potassium channel (HIR) gene to 22q13.1" GENOMICS, ACADEMIC PRESS, SAN DIEGO, US, vol. 26, no. 3, 10 April 1995 (1995-04-10), pages 625-629, XP004828686 ISSN: 0888-7543
- PRÜSS HARALD ET AL: "Kir2 potassium channels in rat striatum are strategically localized to control basal ganglia function." BRAIN RESEARCH. MOLECULAR BRAIN RESEARCH. 20 FEB 2003, vol. 110, no. 2, 20 February 2003 (2003-02-20), pages 203-219, XP002343959 ISSN: 0169-328X
- LEE K H: "Proteomics: a technology-driven and technology-limited discovery science" TRENDS IN BIOTECHNOLOGY, ELSEVIER, AMSTERDAM,, GB, vol. 19, no. 6, 1 June 2001 (2001-06-01), pages 217-222, XP004239791 ISSN: 0167-7799
- SIMPSON R J ET AL: "Cancer proteomics: from signaling networks to tumor markers" TRENDS IN BIOTECHNOLOGY, ELSEVIER PUBLICATIONS, CAMBRIDGE, GB, vol. 19, 1 October 2001 (2001-10-01), pages S40-S48, XP004512844 ISSN: 0167-7799

## Description

The invention relates to the field of diagnosing schizophrenia.

In particular, the invention relates to a method for diagnosing schizophrenia in a subject comprising assessing the level or the expression level of at least one of the following genes or proteins: Kir2.3 or DRD2 or a gene encoding Kir2.3 or DRD2 in peripheral blood lymphocytes (PBL) taken from said subject. The disclosure further relates to agents and uses thereof, said agents specifically binding to said proteins or nucleic acids encoding them, diagnostic kits and screening methods.

### BACKGROUND ART

Schizophrenia is a severe neuropsychiatric disorder characterized by disturbances in mental, emotional and motor processes⁵ This complex and devastating disease affects approximately 1 % of the human population.

There is an abundance of data evidencing that there is a genetic linkage of schizophrenia and that expression pattern of certain genes is altered in patients with schizophrenia. Intensive studies have been carried out to find appropriate genetic markers allowing diagnosis of schizophrenia either before development of symptoms or with an increased reliability. In general, many genes or proteins said to be a marker in schizophrenia were and, in part, are still poorly characterized or there is no convincing evidence that there is significantly different level of expression between healthy and affected individuals.

In WO 0144269 (Johnston-Wilson et al.) brain protein markers for various psychiatric and neurological disorders, including schizophrenia, are taught. Said marker proteins have been identified by 2-D (two dimensional) gel electrophoresis.

In EP 1 348963 A1 (Cochran et al.) it is described that the gene of the voltage-gated potassium channel 3.1 (Kv3.1) α-subunit shows altered expression in schizophrenia and diagnostic and screening methods, based on this finding, are disclosed. To present inventor's knowledge this is the only report on the relation of a potassium channel protein and schizophrenia.

A useful animal model for studying Kir2.3 is described in WO02400661, wherein transgenic mice containing Kir2.3 potassium channel gene disruptions are described. However, no specific mention of use of said mice for studying schizoprenia was found in WO02400661.

One of the hypotheses explaining the nature of the disease is based on dysfunction of dopaminergic system, especially an increased occupancy of D2 subclass of dopamine receptors (DR) by dopamine in schizophrenic patients.⁶ Today antipsychotic drugs used in treatment of schizophrenia act as antagonists of D2 subclass of DRS.⁷ In US 4931270 (Horn et al.) methods and labelled compounds are provided to detect altered distribution of dopamine D2 receptors in schizophrenia.

Expression of neurotransmitters, neuropeptides and D2 subclass of dopamine receptor genes (D₂, D₃, D₄ subtypes) in immune cells, mainly in peripheral blood lymphocytes (PBL), has been reported earlier by several authors.^{1,4,8,9,10} It has been suggested, that they may reflect the status of corresponding brain receptors. In recent years, D₃ subtypes of D2 subclass of dopamine receptor genes (DRD3) were found to be overexpressed in schizophrenic PBL and proposed to be a useful peripheral marker for identification and follow-up of schizophrenia.^{3, 11} The same laboratory reported that α7 nicotinic acetylcholine receptor (α7AChR) gene was also differentially expressed in schizophrenic patients, since in affected PBL a decreased mRNA level of α7AChR had been detected.¹²

Since dopamine interacts directly with DRD3 and DRD2 on T cells and activate integrin mediated T cell adhesion, overexpression of these genes in T cells can serve not only as a passive diagnostic marker but can reflect a dynamic functional interaction.¹³ However, the authors fail to provide any evidence in respect of DRD2 whereas admit that in other diseases as well as during human development the regulation of the two receptors are quite different.

In fact, there is no direct relationship between expression status of either DRD3 or DRD2 receptors in blood cells, e.g. lymphocytes and in the brain has shown. Though it is rather accepted that DRD2 is upregulated in the brain of schizophrenics, there is no indication in the art that DRD2 receptor mRNA level is increased in peripheral blood cells of these patients. In fact, there is a certain doubt whether DRD2 could be detected in lymphocites at all [Ilani T, Ben-Shachar D, Strous RD, Mazor M, Sheinkman A, Kotler M, Fuchs S., "A peripheral marker for schizophrenia: Increased levels of D3 dopamine receptor mRNA in blood lymphocytes" Proc Natl Acad Sci U S A. 2001 98(2):625-8; WO 02/14547].

In recent years fast increase of sequence data and new molecular methods developed to analyze gene expression profile either in high throughput format (microarray technology) or by more precise methods (e.g. quantitative real-time PCR) promise an effective search for new markers.

In EP 1 132 483 A2 (Nawa et al.) a DNA micro-array is used as a tool for finding proteins differentially expressed in schizophrenia. Though a large number of proteins are assumed to be appropriate markers, no convincing evidence is shown in the description.

In WO 0136473 (Vogeli et al.), an extensive study of a large number of putative G-protein coupled receptors is described. Upon expression analysis, a few of them, identified only by their sequence, were proposed to be related to schizophrenia. However, no direct evidence appears to have been provided.

A microarray approach has been utilized in the finding of a decreased expression of G-protein signalling 4 (RGS4) in patients of schizophrenia, as taught in WO 0216653. Vawter et al. screened the PBLs of five individual family members with schizophrenia with a comparison of unaffected individuals to identify differentially expressed genes using microarray of 1128 brain related genes but neither DRD2 nor Kir2.3 were presented on that microarray.³⁵

In WO2004/082570 the use of DRD2 in a number of neurological diseases in mentioned. However, no specific suggestion can be found in relation to schizophrenia.

Though a number of possibly suitable marker genes are known in the prior art, there is still a need to identify further genes which are applicable in the diagnosis of schizophrenia or, if used in combination with known markers, enhance the reliability of existing methods.

Despite extensive studies no suggestion could be found in the prior art that a gene of an inwardly rectifying potassium channel might be a marker of schizophrenia.

Moreover, to the best of inventor's knowledge, in the prior art the use of the gene of DRD2 as a specific diagnostic marker in schizophrenic PBLs has not been not suggested.

Moreover, it has not been known that said genes or the proteins encoded by them, could be peripheral molecular markers in schizophrenia.

The object of the present invention is to provide further diagnostic markers useful in methods for diagnosing schizophrenia.

Present inventors applied both high throughput microarray analysis to pre-scan, and quantitative real-time PCR technique to identify multiple genes expressed differentially in schizophrenic PBL. Genes for DRD2 and Kir2.3 were found to be overexpressed in microarray study. Two genes from DNA microarray results were confirmed by using SybrGreen and TaqMan probes in quantitative real-time PCR. Since neither affected nor non-affected individuals were under antipsychotic or other medication treatment, elevated mRNA levels of DRD2 and Kir2.3 reflect the disorder itself, not the effect of medication. Combination of multiple peripheral molecular genetic markers would be extremely helpful for precise early diagnosis, follow-up of schizophrenia and in decision of effective therapy.

### BRIEF DESCRIPTION OF THE INVENTION

The invention relates to a method for diagnosing schizophrenia in a subject comprising
- assessing the gene expression level of at least one of the following genes:
   a gene encoding Kir2.3 (inwardly rectifying potassium channel 2.3) and/or
   a gene encoding DRD2 (dopamine D2 receptor)
   in peripheral blood lymphocytes taken from said subject and
- comparing the expression level in the biological sample to a baseline expression level(s) of the respective gene(s) typical of healthy subjects or in a healthy subject or to a predetermined control baseline expression level,
wherein a higher gene expression level in the biological sample is considered as an indication of the fact that the subject suffers from or is susceptible to schizophrenia.

Preferably, the method of the invention comprises assessing the expression level of at least a gene encoding Kir2.3. More preferably, level of a gene encoding DRD2 is also assessed.

In a preferred embodiment assessment of the expression level comprises hybridization of a hybridization probe to a nucleic acid obtained from the PBL sample, said nucleic having a sequence
of a gene encoding Kir2.3 and/or
of a gene encoding DRD2 or
a part thereof
or a sequence complementer to any of the above.

Preferably, in the method of the invention a hybridization probe is hybridized to at least a nucleic acid having the sequence of a gene encoding Kir2.3. In a preferred embodiment a further probe is hybridized to a nucleic acid having the sequence of a gene encoding DRD2.

Preferably, the nucleic acid obtained from the biological sample is mRNA or cDNA.

In a preferred embodiment, the expression level is determined by microarray analysis.

Preferably, the expression level is determined by a PCR method, preferably a real time PCR method, more preferably a quantitative real time PCR method.

The biological sample comprises peripheral blood cells, preferably lymphocites, and the gene expression level is assessed in these cells.

In a further embodiment, the invention relates to a method for diagnosing schizophrenia in a subject comprising
- assessing the level of at least one of the following proteins:
   Kir2.3 and/or
   DRD2
   in peripheral blood lymphocytes taken from said subject and
- comparing the level of the protein(s) in the biological sample to a baseline level value of the respective protein typical in healthy subjects or in a healthy subject or to a predetermined control baseline level,
wherein a higher level in the biological sample is considered as an indication of the fact that the subject suffers in or is susceptible to schizophrenia.

Preferably, at least the level of Kir2.3 is assessed. More preferably, at least the level of both Kir2.3 and DRD2 are assessed.

In a preferred embodiment, assessing the level of the protein comprises determination of the activity or the concentration of the protein.

The biological sample isolated from the subject comprises peripheral blood cells, preferably lymphocites, and the activity and/or concentration of the protein(s) is assessed in these cells.

In a further aspect the invention relates to a use of an agent capable of specifically binding to at least one of the following:
Kir2.3 or a nucleic acid encoding the same and/or
DRD2 or a nucleic acid encoding the same
for diagnosing schizophrenia.

Preferably, the agent is capable of specifically binding to at least Kir2.3 or a nucleic acid encoding the same. More preferably, both an agent capable of specifically binding to Kir2.3 or a nucleic acid encoding it and an agent capable of specifically binding DRD2 or a nucleic acid encoding it are used.

In a preferred embodiment, the agent is an oligonucleotide specifically hybridizing to any of the nucleic acids encoding DRD2 or Kir2.3 or a complementer or a part thereof. The oligonucleotide is preferably a probe having a label. The label is preferably a fluorescent label or a radioactive label. Highly preferably, the probe comprises a fluorescent label and a quencher.

In a preferred embodiment the agent is an oligonucleotide primer useful in PCR.

In a further preferred embodiment the agent is a molecule specifically binding to at least one of the following:
Kir2.3 and/or
DRD2.

Preferably, the agent is an antibody, more preferably a monoclonal antibody.

The present disclosure further relates to a diagnostic kit for diagnosing schizophrenia in a subject comprising
at least one agent capable of specifically binding to at least one of the following:
Kir2.3 or a nucleic acid encoding the same, and/or
DRD2 or a nucleic acid encoding the same and
means for producing a detectable signal upon specific binding of the agent.

Preferably, the kit comprises at least an agent capable of specifically binding to Kir2.3 or a nucleic acid encoding it. Preferably, the kit also comprises an agent capable of specifically binding to DRD2 or a nucleic acid encoding it.

Preferably the means producing a detectable signal comprises a label, preferably an enzymatic label, a fluorescent label or a radioactive label.

Preferably the agent is a probe comprising an oligonucleotide specifically hybridizing to any of the nucleic acids or a complementer or a part thereof. The probe may comprise an oligonucleotide and a label bound thereto. Preferably the label is a fluorescent label, and preferably the probe comprises a fluorescent label and a quencher.

Peferably the probe is an oligonucleotide and the label is an intercalating agent, e.g. SYBR-green or ethidium bromide.

Preferably, the diagnostic kit further comprises at least one oligonucleotide primer pair useful in amplifying at least one of the nucleic acids.

Preferably, in the diagnostic kit the agent is a molecule specifically binding to at least one of the following proteins:
Kir2.3 and/or
DRD2.

Preferably, the agent is an antibody, preferably a monoclonal antibody.

In the kit the means for detection of the specific binding can be a label bound to the antibody or a further labelled antibody capable of binding to the first antibody.

In a further aspect the invention relates to a use of an oligonucleotide probe for diagnosing schizophrenia in a subject by assessing gene expression level of a gene encoding Kir2.3 in peripheral blood lymphocytes taken from said subject, said probe comprising and oligonucleotide capable of specifically hybridizing to any of the nucleic acids encoding
Kir2.3 and/or DRD2
or a complementer or a part thereof.

Preferably the probe according to the invention comprises a fluorescent label, and preferably a quencher, bound to the oligonucleotide.

In a further aspect the present disclosure relates to a method for screening for a potential therapeutic agent that modulates symptoms of schizophrenia comprising contacting a candidate compound with at least one of the following proteins or nucleic acids:
Kir2.3 or a nucleic acid encoding it and/or
DRD2 or a nucleic acid encoding it
and assessing a the effect of the compound,
wherein a compound effecting the function of any of the said nucleic acids or proteins is considered as a potential therapeutic agent that modulates symptoms of schizophrenia.

Preferably, in the method a candidate compound is contacted at least with Kir2.3 or a nucleic acid encoding it. In a preferred embodiment the candidate compound is also contacted with DRD2 or a nucleic acid encoding it.

Preferably, the candidate compound is contacted with at least one of the following:
Kir2.3 and/or
DRD2,
and assessing the effect of the compound on the activity of the respective protein,
wherein a compound decreasing the activity is considered as a potential therapeutic agent in schizophrenia.

According to a preferred method the candidate compound is contacted with at least one of the following:
a nucleic acid encoding Kir2.3 and/or
a nucleic acid encoding DRD2
in a cell expressing said nucleic acid(s),
and assessing and evaluating the effect of the compound on the expression level of said nucleic acid(s),
wherein a compound decreasing the expression level is evaluated as a potential therapeutic agent in schizophrenia.

The candidate compound may be administered to an animal model of schizophrenia overexpressing at least one of the following:
Kir2.3 or a nucleic acid encoding it and/or
DRD2 or a nucleic acid encoding it
and assessing the effect of the compound on symptoms associated with schizofrenia.

Preferably, in the animal model at least Kir2.3 or a gene encoding it is overexpressed. More preferably, both Kir2.3 and DRD2, or genes encoding them are overexpressed.

### DEFINITIONS

A "subject" is an individual person or animal. A subject may be a patient who has or may have or is suspected to have a given disorder, or may be a healthy person or animal who optionally can be a control.

A "biological sample" includes any body fluid or tissue obtained from a subject and optionally processed or digested or prepared for study, and containing a protein marker and/or nucleic acid(s) encoding the marker and/or substrates for the protein and/or products produced by the marker. Moreover, a biological sample may be a cell culture, e.g. a primary cell culture obtained by culturing cells of the patient.

A "nucleic acid obtained from a biological sample" is a given nucleic acid which is a component of the sample but which is made available for further studies, e.g. by processing the sample, e.g. by purifying it to certain extent, or by isolating said nucleic acid.

An "immune cell" is a cell which is a descendent of a bone marrow stem cell. Preferred immune cells are cells of myeloid or lymphoid origin. Immune cells are e.g. lymphocytes, natural killer (NK) cells, leukocytes, granulocytes, monocytes, e.g. phagocytes etc.

"Isolation" of a given material means changing its original environment by Man.

A "probe" is a compound capable of reacting with or binding to a biological molecule to be studied, i.e. to the target molecule. Preferably, a probe is designed to contribute to the emission of signals upon reaction with or binding to the said biological molecule.

Preferred type probes for detection of nucleic acids are probes comprising an oligonucleotide (oligonucleotide probes), e.g. fluorescent probes. A highly preferred type of probes contain a fluorescent dye and a quencher capable of fluorescence resonance energy transfer if the probe is not bound to the target molecule (e.g. TaqMan type probes). Special types of such probes are e.g. molecular beacons.

The "expression level" of a gene relates to any measure of the expression allowing quantitative or semi-quantitative assessment or estimation thereof suitable for comparison of expression levels in various samples or subjects. Similarly, the "level" of a protein relates to any measure of the concentration, quantity, effect or activity of the protein allowing quantitative or semi-quantitative assessment or estimation thereof suitable for comparison of levels in various samples or subjects.

"Specific hybridization" relates to binding of a poly or oligonucleotide to an other due to sequence similarity under defined conditions. In a preferred embodiment stringent conditions are applied. Under stringent condition any stringent conditions defined in usual manuals or textbooks in respect of hybridization of probes can be applied. For example, hybridization conditions as defined in the Examples can be applied. Alternatively, As an example, stringent conditions are e.g 65°C in a buffer containing 1 mM EDTA, 0,5 M NaHPO₄, (pH 7,2), 7% (w/v) SDS. Preferably said binding is detectable e.g. by usual means for detecting double stranded DNA.

"DRD2" (dopamine D2 receptor) relates to the D2 subtype of the dopamine receptor. This G-protein coupled receptor inhibits adenyl cyclase activity.

Amino acid sequence of DRD2 protein is given e.g. in PubMed Protein Database at the following Entries: BC021195.2, P14416, NP_057658

Nucleotide sequence of a nucleic acid (e.g. mRNA or cDNA) is given e.g. in PubMed Nucleic Acid Database at the following Entries:X51645, M30625 and NM016574.

Moreover, examples for DRD2 receptor protein and nucleic acids coding therefore are given in the following publications: Strausberg, R.L. et al (2002)³⁶, Araki, K. at al. (1992)³⁷, Dearry, A. (1991)³⁸, Monsma, F. J. (1989) ^{39.} Dal Toso, R. (1989)⁴⁰, Grandy, D. K. (1989)⁴¹ and in WO 2004/082570.

"Kir2.3" (inwardly rectifying potassium channel 2.3) is an integral membrane protein controlled by G proteins and a member of inwardly rectifying K+ channels, which have a greater tendency to allow potassium to flow into the cell rather than out of it. This asymmetry in potassium ion conductance plays a key role in the excitability of muscle cells and neurons. Voltage dependence of Kir2.3 is regulated by the concentration of extracellular potassium; as external potassium is raised, the voltage range of the channel opening shifts to more positive voltages.

Examples for amino acid sequence of the protein are given in Entrez Protein Database e.g. at the following Entries: P52190, NP_690607, NP_004972, Q64198, P52189, P48050, AAG17049, AAG53738

Examples for nucleic acid sequences encoding Kir2.3 are given in Entrez Nucleotides Database e.g. at the following Entries: mRNA: NM_152868, NM_004981 (human); cDNS: AF187874, AF183917 (domestic guinea pig; *Cavia porcellus*)
Moreover, examples for Kir2.3 receptor and nucleic acids coding therefor are given in the following publications: Perier, F. (1994)⁴², Tang, W. (1994)⁴³, Makhina, E. N. (1994)⁴⁴, Budarf, M. L. (1995)⁴⁵.

If a "part" of a protein or of a nucleic acid encoding it or of a gene is mentioned herein it is understood that said part has an essential feature of the said protein or nucleic acid or gene allowing to obtain the same result as if the whole molecule would be used. For example, a part of a protein has the same function or, if the detection of said protein is mentioned, a part of the protein can be detected by the same means. For example, it has the same structural element, e.g. a domain or an epitope characteristic to the whole protein.

If a part of a nucleic acid or a gene is mentioned it must have a sufficient length so as to have a sequence motif unambiguously specific to the whole nucleic acid or gene. For example, under stringent conditions, the same probe can be hybridized to the whole molecule as to the appropriate part thereof.

### BRIEF DESCRIPTION OF THE FIGURES

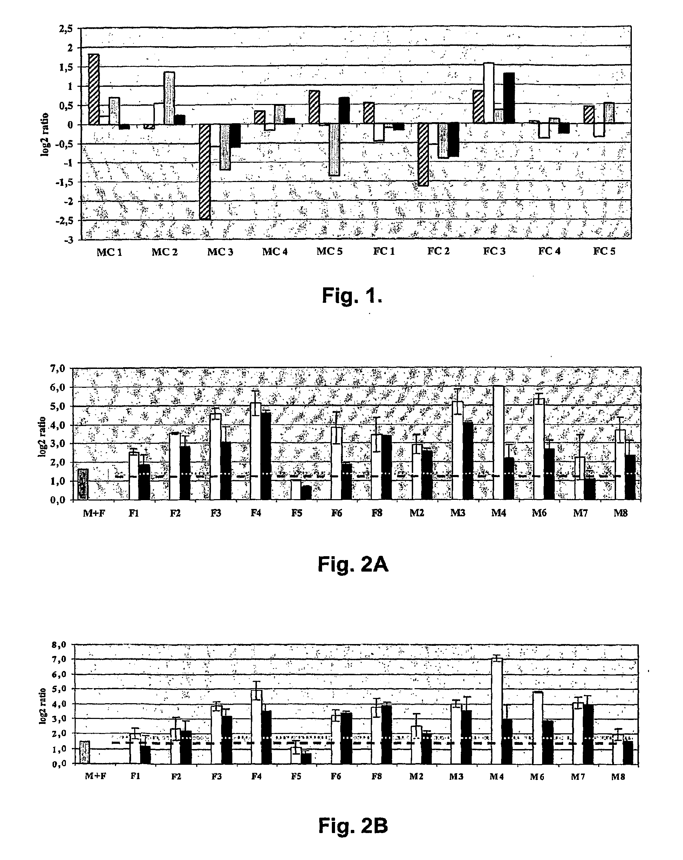

**Figure 1** shows the variation of relative expression level of DRD2 and Kir2.3 genes in non-affected control samples. Relative expression levels are plotted as log₂ ratios on Y-axis. Dashed bars and white bars represent Kir2.3 gene expression levels analyzed by SybrGreen and TaqMan protocols, respectively. Grey and black bars represent the DRD2 gene expression level analyzed by SybrGreen and TaqMan protocols, respectively. (In the figure the meaning of MC is male control; the meaning of FC is female control.
On **Figure 2** the relative expression levels of DRD2 **(A)** and Kir2.3 **(B)** genes in schizophrenic samples are indicated. Ratios are shown as log₂ ratios on Y-axis. White and black bars represent data analyzed by QRT-PCR using SybrGreen dye and TaqMan protocols, respectively. Grey bars show the relative expression level of DRD2 and Kir2.3 gene analyzed by microarray method. **(A)** White dotted line at (log₂radio=1.4) and black dashed line at (log₂ratio=1.3) show the background expression level of DRD2. **(B)** White dotted line at (log₂ratio=1.8), and black dashed line at (log₂ratio=1.5) show the background expression level of Kir2.3. Calculation the value of background relative expression level is based on the variation of relative expression level of DRD2 and Kir2.3 genes in non-affected control samples. (M, male patient; F, female patient).

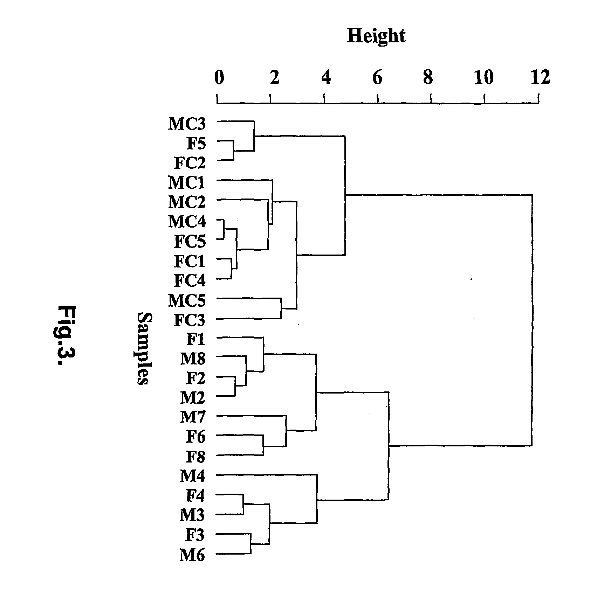

**Figure 3****.** Hierarchical clustering based on expression dataset clearly separates into two main clusters: the non-affected control and affected samples with the evident exception of schizophrenic sample F5. (MC, male control; FC, female control; M, male patient; F, female patient)

### DETAILED DESCRIPTION OF THE INVENTION

Below certain embodiments of the invention are illustrated in more detail by way of examples. It is to be understood that the scope of the invention is not restricted to these examples.

The invention pertains to novel biochemical methods for diagnosing schizophrenia and is based on the finding that genes encoding the dopamine D2 receptor DRD2 and the inwardly rectifying potassium channel Kir2.3 are upregulated in PBL of patients of schizophrenia.

DRD2 belongs to the D2 subclass of DRs and is coupled to G-protein with an inhibiting effect of adenyl cyclase activity.²⁷ Receptor-activated G-proteins can either activate (Kir3.1) or inactivate inwardly rectifying potassium channels (Kir2.3),^{28,29} Several different potassium channels are known to be involved in electrical signaling in the nervous system. Kir2.3 is a member of Kir2 family of constitutively active inwardly rectifier K⁺ channels carrying large inward and small outward potassium current.³⁰ This asymmetry in potassium ion conductance plays a key role in the excitability of muscle cells and neurons. DA (dopamine) through D2 subclass of DRs directly activates an IAP (Islet activating protein, pertussis toxin) -sensitive G protein coupled with inward rectifier potassium channels in rat substantia nigra neurones.³¹ Lavine *et al*. demonstrated that DRD2 and another inwardly rectifying potassium channel Kir3, form a stable functional complex both *in vitro* and *in vivo*.³² Though the present invention is by no means to be limited by any theory behind, these results suggest a strong association between DRs and inwardly rectifying potassium channels.

In one embodiment of the invention the expression level of either the gene encoding DRD2 or the gene encoding Kir2.3 or both is assessed in a PBL sample taken from a subject. The expression level determined in the biological sample is compared to a baseline expression level of the respective gene, typical of healthy subjects. If the gene expression level in the PBL sample is higher than the baseline level, this finding is considered as an indication of the fact that the subject suffers in or is susceptible to schizophrenia.

In an other embodiment the level of either a DRD2 protein or a Kir2.3 protein is assessed in a PBL sample taken from a subject. The level of the protein(s) in the PBL sample is compared to a baseline level value of the respective protein typical in healthy subjects. If the level in the biological sample is higher than the baseline level the finding is considered as an indication of the fact that the subject suffers in or is susceptible to schizophrenia.

It is to be emphasized that other markers of schizophrenia have been known in the art, examples for which are listed in the introductory part of the present description. The present novel markers also can be used together with the known markers improving the reliability of diagnosis.

The baseline expression level can be the expression level of the same gene in a healthy, i.e. non-schizophrenic, subject. More preferably the baseline level is defined as an average of expression levels of a reference group of healthy subjects. It may be advisable if the reference group is selected carefully an the subjects in this group and the subject to be diagnosed advantageously match each other in respect of certain characteristics, e.g. race, age, sex etc, though our results suggest that variations within healthy subjects are usually smaller than the differences between healthy and schizophrenic subjects. The baseline level is expediently defined relative to a suitable control, e.g. an internal reference control. Alternatively, for a more general use the reference group of subjects should be sufficiently diverse in terms of age, sex, social status, geographical distribution, previous drug and medical histories, etc. and of sufficient size to provide a meaningful statistical value. In this way expression of the diagnostic marker genes or proteins of the invention is measured in the set of samples representing the "normal" population. Baseline level value is calculated from a statistic of these results.

The skilled person has known for a long time a number of methods for quantitative detection of proteins levels or of gene expression levels in a sample. All these methods may be applicable in the invention.

In one embodiment of such a detection scheme, a cDNA molecule is synthesized from an RNA molecule of interest (e.g. by reverse transcription of the RNA molecule into cDNA). A sequence within the cDNA is then used as the template for a nucleic acid amplification reaction, such as a PCR amplification reaction, or the like. The nucleic acid reagents used as primers in the reverse transcription and nucleic acid amplification steps of this method are chosen from DRD2 and Kir2.3 gene nucleic acid reagents. Those skilled in the art are familiar with techniques for designing and obtaining suitable primers (see the Examples).

Additionally, it is possible to perform such gene expression assays "*in situ*", i.e. directly upon tissue sections (fixed and/or frozen) of patient tissue obtained from biopsies or resections, such that no nucleic acid purification is necessary. Nucleic acid reagents may be used as probes and/or primers for such *in situ* procedures. Alternatively, if a sufficient quantity of the appropriate cells can be obtained, standard Northern analysis can be performed to determine the level of mRNA expression of the marker genes.

However, recently, certain relatively new method have gained widespread applicability.

As demonstrated by the Examples below, one technique for establishing baseline levels may involve real time quantitative PCR (RT-PCR). A variety of RT-PCR methods are known by the skilled person such as the methods described or referred in the Real Time PCR Special Issue of the METHODS Journal (Elsevier, Dec 2001, Volume 25, Issue 4. pages 383 to 481).

According to another embodiment of the present invention, when protein levels are assessed, antibodies which specifically recognize either DRD2 or Kir2.3 are produced. The antibodies are used alone or in combination. The level of binding of antibodies which specifically recognize the proteins will be higher in schizophrenic individuals than in normal individuals. An antibody against the Kir2.3. protein is described e.g. by Noam et al (1996)⁴⁶.

In order to normalize the level of binding to the total amount of proteins present in the tissue, the binding of the specific antibodies can be expressed relative to the binding of an antibody to a reference protein equally expressed in normal and diseased subjects.

In an alternate embodiment of the invention, the determination of the amount of modified and unmodified receptors can be made by isolation, e.g. partial isolation of the receptor protein or the potassium channel protein itself. Effective methods for isolation are gel electrophoresis, e.g. two dimensional gel electrophoresis, or isoelectric focusing techniques. A combination of these techniques with blotting techniques or other methods of immunological detection may provide an efficient tool for assessing the concentrations of quantities of the proteins in given samples. The isolated or partially purified proteins may be subjected to analysis of peptide fragments, obtained e.g. by proteolitic enzymes, which carry special properties e.g. may contain a site suitable for specific chemical modification.

According to an alternative embodiment the activity of the marker receptor protein DRD2 or the potassium channel Kir2.3 is measures as a value indicative of the level of said protein. Methods for determination of inward rectifier K+ currents and further methods to detect Kir2.3 are described e.g. in Noam et al (1996)⁴⁶ and Bannister et al. (1999)⁴⁷ and publications referred therein.

When the level of the marker protein or the marker gene is obtained and compared to the baseline level a certain threshold value can be determined above which the levels in samples from the diagnosed subjects are accepted as indicative of schizophrenia. This threshold value may depend on a variety of factors, including the measurement method, reliability of the baseline level, statistical (mathematical) considerations and the aim of the diagnosis (i.e. that the development of the disease has to be decided with certainty or only a susceptibility is to be ascertained) etc. As an extreme value, the baseline may coincide with the threshold value. Determination of levels above this threshold value may be indicative of the severity of the disease.

Advantageously, when a concrete method is optimized, a set of diseased subjects is also assayed to determine validity of the test by comparing results of the diseased sample to those of the normal sample.

Diagnostic kits are well known in the art. In particular, a number of diagnostic kits are available and are described in the prior art cited in the present description. Any of these kits can be used as analogue of the kits of the present disclosure taking into account the specific features of the invention taught herein.

### EXAMPLES

### Materials and Methods

### Patients

Patients were recruited from the inpatient unit of the Department of Psychiatry and informed consent was obtained after explanation of the study. Each patient went through profound medical examination including physical and neurological examination and routine laboratory urine drug tests also. Each of them received the Mini International Neuropsychiatric Interview Plus (MINI Plus)²¹, and was diagnosed with schizophrenia or schizophreniform psychosis according to DSM-IV.²² Psychiatric symptoms were assessed using the Positive and Negative Syndrome Scale (PANSS)²³, the Clinical Global Impression (CGI) and the Global Assessment of Functioning (GAF) scale.²² Clinical ratings were performed by two trained psychiatrists. The study was conducted in accordance with Declaration of Helsinki. Demographical and medical data are detailed in (Table 2.) 7 female and 6 male drug-naive or drug-free schizophrenic patients and 10 healthy control individuals were analyzed.

### Isolation of PBL and RNA from schizophrenic patients and control individuals

Lymphocytes fraction of 10 ml of total blood (collected in EDTA-treated tubes) was washed 3 times with red blood lysis buffer (5 mM MgCl₂, 10 mM NaCl, 10 mM Tris-HCl, pH 7.0, Promega, Madison, WI, USA) to lyse erythrocytes. RNA isolation from clear pellet of lymphocytes was carried out by the RNA isolation kit of Macherey-Nagel (Macherey-Nagel, Düren, Germany) according the manufacturers' instruction. RNA samples were stored at -80°C in the presence 30 U of Prime RNAse inhibitor (Eppendorf, Hamburg, Germany). The quality and quantity of isolated RNA was checked by electrophoresis and spectrophotometry (NanoDrop, Rockland, DE, USA) respectively.

### Microarrays, probe preparations and hybridizations

3200 amplified cDNA inserts from human lymphocyte cDNA Matchmaker library (Clontech, BD Biosciences Franklin Lakes, NJ, USA) (1200 clones) and 2000 sequence verified clones from mixed human library in pBluescript SK II (-) plasmid (New England Biolabs, Hertfordshire, England) were amplified by PCR with plasmid specific primers and purified with MultiScreen-PCR plate (Millipore, Billerica, MA, USA), resuspended in 50% dimethyl sulfoxide/water, and arrayed on FMB cDNA slides (Full Moon BioSystems, Sunnyvale, CA, USA) using a MicroGrid Total Array System (BioRobotics, Cambridge, UK) spotter. Post-processing and blocking of the microarrays were performed as described previously.²⁴ For probe preparation, 2 µg of total RNA (a pool of 14 schizophrenic patients) was reverse transcribed using poly-dT primed Genisphere Expression Array 350 Detection Kit system (Genisphrere, Hatfield, PA, USA) according the manufacturer instruction. cDNA with capture sequence was hybridized onto human cDNA microarray containing 3200 human cDNA clones. Both the first step cDNA hybridization and the second step capture reagent hybridization were carried out in a Ventana hybridization station (Ventana Discovery, Tucson, AZ, USA) by using the "antibody" protocol. First hybridization was performed at 40 °C for 6 hours in "Chiphyb" hybridization buffer (Ventana), then 2.5 µl of each Cy5 and Cy3 capture reagents were added to the slides in 200 µl "Ribohyb" hybridization buffer (Ventana) and incubated at 42 °C for 2 hours. After hybridization, the slides were washed in 0.2X SSC twice at RT for 10 min, then dried and scanned. Scanning and data analysis were done as describe earlier.²⁵

### Real-Time PCR

For QRT-PCR 1 µg of total RNA from individual schizophrenic patients and control individuals were reverse transcribed using SuperScript II RNase H⁻ Reverse transcriptase (Invitrogen, Carlsbad, CA, USA). The reactions were carried out in 20 µl of final volume in the presence of 3.5 µM Random Hexamer Primer, 1x First-Strand Buffer (250 mM Tris-HCl (pH 8.3) 375 mM KCI, 15 mM MgCl₂), 500 nM dNTP mix (500 nM of each) 10 mM DTT, 40 U Prime RNAse inhibitor (Eppendorf) and 200 U of SuperScript II RNase H⁻ Reverse transcriptase (Invitrogen) at 42°C for 2 hours. RNA and random primer mixture was denatured at 65°C for 5 minutes before RT reaction. The enzyme reaction was terminated by inactivating the reverse transcriptase at 70°C for 15 minutes. The final volume of RT reaction was diluted 4 times. 1 µl of the diluted reaction mix was used for QRT-PCR using either SybrGreen dye or TaqMan probes. Reactions using SybrGreen protocol were done with ABsolute QPCR SYBR Green mix (ABGene, Surrey, UK) in RotorGene real-time Q-PCR machine (Corbett Research, Mortlake, Australia) according to manufacturer instruction at final concentration of 150 nM primers under following conditions; 15 min at 95°C, and 45 cycles of 95°C for 15 sec, 60°C for 25 sec and 72°C for 25 sec. Fluorescence intensity of SybrGreen dye was detected after each amplification step. Melt temperature analysis was done after each reaction to check the quality of the reaction. PCR reactions with TaqMan probes were done using ABsolute QPCR mix (ABGene) according to manufacturer instruction in the presence of 300 nM of primers and 250 nM of TaqMan probes under the following conditions; 15 min at 95°C, and 45 cycles of 95°C for 15 sec, 60°C for 1 min. Intensity of fluorescein dye (FAM) was detected after each cycle. PCR primers and TaqMan probes were designed using the Primer Express Software (Applied Biosystems, Foster City, CA, USA). Primers and TaqMan probes used in this study are summarized in Table 1. Dual-labeled probes were prepared by Bioneer (Daejeon, Korea) with Fluorescein dye at 5' end and Dabsyl dye at 3' end. The quality of the primers was verified by MS analysis provided by Bioneer. Non-template control sample was used for each PCR run to check the genomic DNA contaminations of cDNA template. Analysis of results was done using Pfaffl method.²⁶ Using this calculation method differences between the amplification efficiencies of reactions can be corrected.

**Table 1**

| **Short name** | **PCR Primers** | **TaqMan Probe** | **Gene Name** | **Acc No.** |
|---|---|---|---|---|
| DRD2 | For CTGCTCATCGCTGTCATCGT | TCGGCAACGTGCTG GTGTGCA | Human D2 dopamine receptor | X51362 X51362- |
| | Rev CTCGCGGGACACAGCC | | | |
| Kir2.3 | For CTTCCCCCACTGACTCTTCAAG | TGCCCTCTTTGCTCT CAGAACCTTGG | Homo sapiens potassium inwardly-rectifying channel (Kir2.3) | NM_004 981 |
| | Rev CCAGCAGTCCAGCCACCTT | | | |
| Actin | For TCACCGAGCGCGGCT | CAGCTTCACCACCAC GGCCGA | Human actin, beta (ACTB), | NM_001 101 |
| | Rev TAATGTCACGCACGATTTCCC | | | |
| HPRT | For TGACACTGGCAAAACAATGCA | CTTTGCTTTCCTTGG TCAGGCAGTATAATC CA | Human hypoxanthine phosphoribosyl-transferase (HPRT) | M31642 |
| | Rev GCTTGCGACCTTGACCATCT | | | |

### Statistical analysis

Unpaired Student's *t* test (two-tailed) was applied to determine the p value. To divide the gene expression dataset into groups of observations that are similar to each other, agglomerative hierarchical clustering was applied. The employed method was the group average method. Euclidean metric (root sum-of-squares of differences) was used to calculate the dissimilarities between observations. The routine is implemented in the R1.8.1 statistical software.

### Results

### Patients and control individuals

We screened 13 drug-naive or drug-free schizophrenic patients (7 female and 6 male, age range 23-67 years, average 34±12) and 10 control individuals (5 male and 5 female, age range 28-65 years, average 38±10) to identify novel peripheral genetic markers of schizophrenia. Demographic data of schizophrenic patients and control individuals are summarized in Table 2. Neither affected nor non-affected individuals were under antipsychotic treatment or other medication.

**Table 2**

| **Patients** | **Status** (duration of drug-free period) | **Age** | **Duration of illness** | **PANSS** | | | | **CGI** | **GAF** | **Medication on date of sampling** | | |
|---|---|---|---|---|---|---|---|---|---|---|---|---|
| | | | | **positive** | **negative** | **global** | Σ | | | **APS** | **ANX** | **other** |
| **Male 2** | F (1 month) | 34 | 3 years | 26 | 25 | 55 | 106 | 4 | 50 | ∅ | ∅ | ∅ |
| **Male 3** | N | 34 | 6 months | 25 | 21 | 36 | 82 | 4 | 40 | ∅ | ∅ | ∅ |
| **Male4** | F (4 months) | 36 | 4 years | 33 | 31 | 65 | 129 | 6 | 25 | ∅ | ∅ | ∅ |
| **Male 6** | N | 25 | 4.5 years | 29 | 36 | 66 | 131 | 6 | 20 | ∅ | ∅ | ∅ |
| **Male 7** | N | 25 | 7 months | 34 | 32 | 74 | 140 | 6 | 20 | ∅ | ∅ | ∅ |
| **Male 8** | N | 24 | 6 months | 14 | 37 | 49 | 100 | 5 | 25 | ∅ | ∅ | ∅ |
| **Female 1** | F (3 months) | 46 | 15 years | 31 | 25 | 58 | 114 | 6 | 30 | ∅ | ∅ | ∅ |
| **Female 2** | F (3 months) | 34 | 14 years | 34 | 40 | 85 | 159 | 7 | 15 | ∅ | ∅ | ∅ |
| **Female 3** | F (1 year) | 67 | 17 years | 29 | 28 | 51 | 108 | 5 | 30 | ∅ | ∅ | ∅ |
| **Female 4** | F (1 year) | 29 | 5 years | 29 | 30 | 54 | 113 | 6 | 30 | ∅ | ∅ | ∅ |
| **Female 5** | N | 27 | 9 months | 40 | 34 | 81 | 155 | 7 | 15 | ∅ | ∅ | ∅ |
| **Female 6** | N | 33 | 8 months | 22 | 23 | 54 | 99 | 4 | 40 | ∅ | ∅ | ∅ |
| **Female 8** | N | 23 | 3 months | 22 | 28 | 62 | 112 | 5 | 30 | ∅ | ∅ | ∅ |

| | | | | | | | | | | | | |
|---|---|---|---|---|---|---|---|---|---|---|---|---|
| N: drug-naive, F: drug-free PANSS: Positive and Negative Syndrome Scale positive: positive syndrome items negative: negative syndrome items global: global syndrome items Σ: PANSS total score CGI: Clinical Global Impression GAF: Global Assessment of Functioning APS: antipsychotic ANX: anxiolytic | | | | | | | | | | | | |

### Pre-screen by cDNA microarray analysis

cDNA microarray analysis was performed in order to pre-screen expression pattern of schizophrenic PBL and to identify potential candidate peripheral marker genes. Genes with altered expression were subjected to further analysis using QRT-PCR with SybrGreen dye and dual labeled TaqMan probes. Total RNA pool of 13 affected male and female patients and 10 control individuals were reverse transcribed and hybridized onto human cDNA microarray containing 3200 human cDNA clones. Positive clones showing more than two-fold change in their expression compared to controls were sequenced. Among others, 2.56±0.26- and 6.75±2.3-fold increase in expression level of DRD2 and Kir2.3 was detected, respectively, in schizophrenic patients compared to healthy individuals (Fig.2). Based on this preliminary data and considering the potential role of these genes in pathogenesis of schizophrenia, expression levels of DRD2 and Kir2.3 genes were subjected to further analysis.

### Reference controls and baseline values

After reverse transcription of 13 individual total RNA samples of affected and 10 non-affected individuals, QRT-PCR was performed on cDNA templates mixed with primers and TaqMan probes specific for DRD2 and Kir2.3 genes. β-actin or hypoxanthine phosphoribosyl-transferase (HPRT) genes were used as internal reference controls. Relative quantification using Ct values and reaction efficiency values was performed to determine the exact ratio between the normalized Ct values of both genes in all individual affected samples compared to the average values calculated from Ct values of the 5-5 sex matched individual control samples.

To determine the variation of DRD2 and Kir2.3 mRNA levels in the non-affected samples, relative expression levels of both genes were calculated. The variation of relative expression level of DRD2 and Kir2.3 genes in control samples is shown in Fig 1. Relative mRNA level of DRD2 and Kir2.3 in non-affected samples were changed in a range of 0.39- to 2.56-fold and 0.18- to 3.54-fold, respectively, using the SybrGreen method and 0.55- and 2.42-fold and 0.67- to 2.92-fold, respectively, using TaqMan probe method. Considering these variations of relative expression level of DRD2 and Kir2.3 genes in non-affected control samples, a background level of relative expression was calculated. Only affected PBLs with relative expression level of DRD2 and Kir2.3 higher than any of the non-affected control were regarded as positives. Using SybrGreen method, ratios above 2.56 and 3.54 were regarded as up-regulated in the case of DRD2 and Kir2.3 genes, respectively. In the case of TaqMan protocol, ratios above 2.42 and 2.92 were regarded as up-regulated in the case of DRD2 and Kir2.3 genes, respectively (Fig. 2, white dotted and black dashed lines).

### Overexpression of the DRD2 gene

DRD2 gene was found to be significantly overexpressed in 6 male and 6 female schizophrenic patients compared to healthy controls in a range of 5.7-fold to 64.7-fold using the SybrGreen method. Relative expression level of DRD2 gene of one female affected PBL (F5) was under the background level. In this case a 2.1-fold overexpression could be detected. The average increase of DRD2 gene expression detected by the SybrGreen method was 21.4±18.5-fold (*p*=0.0019) (Fig2, A, white bars). Using TaqMan probe, 6 female and 5 male schizophrenic PBLs showed increased mRNA level of DRD2 gene (3.7- to 24.1-fold). Relative expression levels of DRD2 of one female and one male affected PBLs (F5, M7) were under the calculated background level (1.6- and 2.2-fold, respectively). The average increase of DRD2 gene expression analyzed by TaqMan protocol was 7.8±6.3-fold (*p*=0.0023) (Fig2A, black bars).

### Overexpression of the Kir2.3 gene

In the case of 6 male and 6 female schizophrenic PBL, the expression level of Kir2.3 gene was also above the background expression level compared to healthy controls. Ratios of mRNA level of Kir2.3 in affected versus non-affected sample were varied in a range of 4- to 133,6-fold using the SybrGreen method. Relative expression level of Kir2.3 gene of one female affected PBL (F5) was under the background value (2.2-fold). The average increase of relative expression level of Kir2.3 analyzed by SybrGreen method was 22±34.7-fold (p=0.0535) (Fig2B, white bars). These changes in expression level were confirmed by the method using dual labeled TaqMan probes as well. 5 female and 6 male schizophrenic PBL showed increased mRNA level of Kir2.3 gene (2.8- to 15.8-fold). In case of 2 female affected PBLs (F1, F5), relative expression levels of Kir2.3 were under the calculated background level (2.4- and 1.6-fold, respectively). The average increase of Kir2.3 gene expression analyzed by TaqMan probe was 8.2±4.8-fold (p=0.0001) (Fig2B, black bars). Differences in expression levels observed by the two different real-time PCR methods could be explained by the more precise and sensitive features of the TaqMan protocol.

### Similarity groups

To determine similarity groups of gene expression dataset, agglomerative hierarchical clustering was applied. The non-affected control and affected samples were correctly classified into two separate clusters with the evident exception of the schizophrenic sample F5. There was no significant differences between relative expression levels of female and male patients (Fig. 3).

Among the genes that were found to be differently expressed in affected PBL by microarray method, up-regulation in expression of two genes, DRD2 and Kir2.3 were confirmed by SybrGreen and TaqMan probes based QRT-PCR. Since neither affected nor non-affected individuals were under antipsychotic or other medication treatment, elevated mRNA levels of DRD2 and Kir2.3 reflect the disorder itself, not the effect of medication. Thus, DRD2 and Kir2.3 can serve as additional peripheral genetic markers for prediction of schizophrenia. High-throughput analysis of already reported molecular markers and genes in combination with the presented ones help the earlier and more precise diagnosis of this disorder. Diagnostic tests based on these results can be developed and help manage schizophrenia from the beginning to the chronic state.

A number of methods useful in the present invention is described in the prior art cited herein.

### References

1. McKenna F, McLaughlin PJ, Lewis BJ, Sibbring GC, Cummerson JA, Bowen-Jones D et al. Dopamine receptor expression on human T- and B-lymphocytes, monocytes, neutrophils, eosinophils and NK cells: a flow cytometric study. J Neuroimmunol 2002; 132: 34-40.
2. Ricci A, Bronzetti E, Felici L, Greco S, Amenta F. Labeling of dopamine D3 and D4 receptor subtypes in human peripheral blood lymphocytes with [3H]7-OH-DPAT: a combined radioligand binding assay and immunochemical study. J Neuroimmunol 1998; 92: 191-5.
3. Ilani T, Ben-Shachar D, Strous RD, Mazor M, Sheinkman A, Kotler M et al. A peripheral marker for schizophrenia: Increased levels of D3 dopamine receptor mRNA in blood lymphocytes. Proc NatI Acad Sci U S A 2001; 98: 625-228.
4. Ricci A, Bronzetti E, Felici L, Tayebati SK, Amenta F. Dopamine D4 receptor in human peripheral blood lymphocytes: a radioligand binding assay study. Neurosci Lett 1997; 229: 130-134.
5. Lewis DA, Lieberman JA. Catching up on schizophrenia: natural history and neurobiology. Neuron 2000; 28:325-34.
6. Abi-Dargham A, Rodenhiser J, Printz D, Zea-Ponce Y, Gil R, Kegeles LS. et al. Increased baseline occupancy of D2 receptors by dopamine in schizophrenia. Proc Natl Acad Sci U S A 2000; 97: 8104-9.
7. Seeman P. Dopamine receptors and the dopamine hypothesis of schizophrenia. Synapse 1987; 1: 133-52.
8. Ricci A, Veglio F and Amenta F. Radioligand binding characterization of putative dopamineD3 receptor in human peripheral blood lymphocytes with [3H] 7-OH-DPAT. J. Neuroimmunol 1995; 58: 139-144.
9. Bondy B, de Jonge S, Pander S, Primbs J, Ackenheil M. Identification of dopamine D4 receptor mRNA in circulating human lymphocytes using nested polymerase chain reaction. J Neuroimmunol 1996; 71: 139-144.
10. Bishopric NH, Cohen HJ, Lefkowitz RJ. Beta adrenergic receptors in lymphocyte subpopulations. J Allergy Clin Immunol 1980; 65: 29-33.
11. Kwak YT, Koo MS, Choi CH, Sunwoo I. Change of dopamine receptor mRNA expression in lymphocyte of schizophrenic patients. BMC Med Genet 2001; 2:3.
12. Perl O, Ilani T, Strous RD, Lapidus R, Fuchs S. The alpha7 nicotinic acetylcholine receptor in schizophrenia: decreased mRNA levels in peripheral blood lymphocytes. FASEB J 2003; 17: 1948-50.
13. Levite M, Chowers Y, Ganor Y, Besser M, Hershkovits R, Cahalon L. Dopamine interacts directly with its D3 and D2 receptors on normal human T cells, and activates beta1 integrin function. Eur J Immunol 2001; 12: 3504-12.
14. Barbanti P, Fabbrini G, Ricci A, Bruno G, Cerbo R, Bronzetti E, Amenta F, Luigi Lenzi G. Reduced density of dopamine D2-like receptors on peripheral blood lymphocytes in Alzheimer's disease. Mech Ageing Dev 2000; 120: 65-75.
15. Mimics K, Middleton FA, Marquez A, Lewis DA, Levitt P. Molecular characterization of schizophrenia viewed by microarray analysis of gene expression in prefrontal cortex. Neuron 2000; 28: 53-67.
16. Mimics K, Middleton FA, Lewis DA, Levitt P. Analysis of complex brain disorders with gene expression microarrays: schizophrenia as a disease of the synapse. Trends Neurosci 2001; 24: 479-86.
17. Mimics K, Middleton FA, Stanwood GD, Lewis DA, Levitt P. Disease-specific changes in regulator of G-protein signaling 4 (RGS4) expression in schizophrenia. Mol Psychiatry 2001; 6: 293-301.
18. Middleton FA, Mimics K, Pierri JN, Lewis DA, Levitt P. Gene expression profiling reveals alterations of specific metabolic pathways in schizophrenia. J Neurosci 2002; 22: 2718-29.
19. Hakak Y, Walker JR, Li C, Wong WH, Davis KL, Buxbaum JD, Haroutunian V, Fienberg AA. Genome-wide expression analysis reveals dysregulation of myelination-related genes in chronic schizophrenia. Proc Natl Acad Sci U S A 2001; 98: 4746-51.
20. Marcotte ER, Srivastava LK, Quirion R. cDNA microarray and proteomic approaches in the study of brain diseases: focus on schizophrenia and Alzheimer's disease. Pharmacol Ther 2003; 100: 63-74.
21. Balázs J, Bitter I. [Study on construct validity of the M.I.N.I. PLUS interview]. Psychiat Hung 2000; 15: 134-44.
22. American Psychiatric Association. DSM-I: Diagnostic and Statistical Manual of Mental Disorders, 4th ed. Washington, DC: American Psychiatric Associaton,1994.
23. Kay SR, Fiszbein A, Opler LA. The positive and negative syndrome scale (PANSS) for schizophrenia. Schizophr Bull. 1987; 13: 261-76.
24. Puskas LG, Hackler L Jr, Kovacs G, Kupihar Z, Zvara A, Micsik T, van Hummelen P. Recovery of cyanine-dye nucleotide triphosphates. Anal Biochem 2002; 305: 279-81.
25. Onody A, Zvara A, Hackler LJ, Vigh L, Ferdinandy P, Puskas LG. Effect of classic preconditioning on the gene expression pattern of rat hearts: a DNA microarray study. FEBS Lett. 2003; 536: 35-40.
26. Pfaffl MW. A new mathematical model for relative quantification in real-time RT-PCR. Nucleic Acids Res 2001; 29: e45.
27. Albert PR, Neve KA, Bunzow JR, Civelli O. Coupling of cloned rat dopamine-D2 receptor to inhibition of adenylyl cyclase and prolactin secretion. J Biochem 1990; 265, 2098-2104.
28. Myeong H, Jeoung D, Kim H, Ha JH, Lee Y, Kim KH et al. Genomic analysis and functional expression of canine dopamine D2 receptor. Gene 2000; 257: 99-107.
29. Cohen NA, Sha Q, Makhina EN, Lopatin AN, Linder ME, Snyder SH, Nichols CG. Inhibition of an inward rectifier potassium channel (Kir2.3) by G-protein betagamma subunits. J Biol Chem 1996; 271: 32301-5.
30. Perier F, Radeke CM, Vandenberg CA. Primary structure and characterization of a small-conductance inwardly rectifying potassium channel from human hippocampus. Proc Natl Acad Sci U S A 1994; 91: 6240-4.
31. Uchida S, Akaike N, Nabekura J. Dopamine activates inward rectifier K+ channel in acutely dissociated rat substantia nigra neurones. Neuropharmacology 2000; 39: 191-201.
32. Lavine N, Ethier N, Oak JN, Pei L, Liu F, Trieu P et al. G protein-coupled receptors form stable complexes with inwardly rectifying potassium channels and adenylyl cyclase. J Biol Chem 2002; 277: 46010-9.
33. Roberts DA, Balderson D, Pickering-Brown SM, Deakin JF, Owen F. The abundance of mRNA for dopamine D2 receptor isoforms in brain tissue from controls and schizophrenics. Brain Res Mol Brain Res 1994; 25: 173-175.
34. Tallerico T, Novak G, Liu IS, Ulpian C, Seeman P. Schizophrenia: elevated mRNA for dopamine D2 (Longer) receptors in frontal cortex. Brain Res Mol Brain Res 2001; 87: 160-165.
35. Vawter MP, Ferran E, Galke B, Cooper K, Bunney WE, Byerley W. Microarray screening of lymphocyte gene expression differences in a multiplex schizophrenia pedigree. Schizophr Res 2004; 67: 41-52.
36. Strausberg, R.L. et al, Generation and initial analysis of more than 15,000 full-length human and mouse cDNA sequences, Proc. Natl. Acad. Sci. U.S.A. 99 (26), 16899-16903 (2002)
37. Araki, K., Kuwano, R., Morii, K., Hayashi, S., Minoshima, S., Shimizu, N., Katagiri, T., Usui, H., Kumanishi, T. and Takahashi, Y., Structure and expression of human and rat D2 dopamine receptor genes, Neurochem. Int. 21 (1), 91-98 (1992)
38. Dearry, A., Falardeau, P., Shores, C. and Caron, M. G., D2 dopamine receptors in the human retina: cloning of cDNA and localization of mRNA, Cell. Mol. Neurobiol. 11 (5), 437-453 (1991)
39. Monsma,F.J. Jr., McVittie,L.D., Gerfen,C.R., Mahan,L.C. and Sibley,D.R. TITLE Multiple D2 dopamine receptors produced by alternative RNA splicing JOURNAL Nature 342 (6252), 926-929 (1989)
40. Dal Toso, R., Sommer, B., Ewert, M., Herb, A., Pritchett, D.B., Bach, A., Shivers, B.D. and Seeburg, P.H., The dopamine D2 receptor: two molecular forms generated by alternative splicing, EMBO J. 8 (13), 4025-4034 (1989)
41. Grandy, D. K., Marchionni, M. A., Makam, H., Stofko, R. E., Alfano, M., Frothingham, L., Fischer, J. B., Burke-Howie, K. J., Bunzow, J. R., Server, A. C. et al., Cloning of the cDNA and gene for a human D2 dopamine receptor, Proc. Nail. Acad. Sci. U.S.A. 86 (24), 9762-9766 (1989)
42. Perier, F., Radeke, C. M. and Vandenberg, C. A., Primary structure and characterization of a small-conductance inwardly rectifying potassium channel from human hippocampus, Proc. Natl. Acad. Sci. U.S.A. 91 (13), 6240-6244 (1994)
43. Tang, W. and Yang, X. C., Cloning a novel human brain inward rectifier potassium channel and its functional expression in Xenopus oocytes, FEBS Left. 348 (3), 239-243 (1994)
44. Makhina, E. N., Kelly, A. J., Lopatin, A. N., Mercer, R. W. and Nichols, C. G., Cloning and expression of a novel human brain inward rectifier potassium channel, J. Biol. Chem. 269 (32), 20468-20474 (1994)
45. Budarf, M. L., Perier, F., Barnoski, B. L., Bell, C. J. and Vandenberg, C. A., Assignment of the human hippocampal inward rectifier potassium channel (HIR) gene to 22q13.1, Genomics 26 (3), 625-629 (1995)
46. Noam A. Cohen, Qun Shan, Elena N. Makhina, Anatoli N. Lopatin, Maurine E. Linder, Solomon H. Snyder and Collin, G. Nichols, Inhibition of an Inward Rectifier Potassium Channel (Kir2.3) by G-proteinβγ Subunits, J. Biol. Chem. 271 (50), 32301-32305 (1996)
47. Bannister JP, Young BA, Sivaprasadarao A, Wray D., Conserved extracellular cysteine residues in the inwardly rec potassium channel Kir2.3 are required for function but not expression in the membrane, FEBS Lett., 24, 458 83): 393-9, (1999).

## Claims

1. A method for diagnosing schizophrenia in a subject comprising
- assessing the gene expression level of at least a gene encoding Kir2.3 (inwardly rectifying potassium channel 2.3) and, preferably a further gene encoding DRD2 (dopamine D2 receptor)
in peripheral blood lymphocytes taken from said subject and
- comparing the expression level in the peripheral blood lymphocytes to a baseline expression level of the respective gene typical of healthy subjects,
wherein a higher gene expression level in the peripheral blood lymphocytes is considered as an indication of the fact that the subject suffers from or is susceptible to schizophrenia.

2. The method of claim 1 wherein assessment of the expression level comprises hybridization of one or more hybridization probe to one or more mRNA obtained from the peripheral blood lymphocytes, said one or more hybridization probe or said one or more mRNA having a sequence
of a gene encoding Kir2.3 and, preferably
if the gene expression level of a gene encoding DRD2 is also assessed, of a gene encoding DRD2 or
of a part thereof
or a sequence complementary to any of the above.

3. The method of claim 1 or claim 2 wherein the expression level is determined by microarray analysis, preferably by a PCR method, preferably a real time PCR method.

4. Use of an agent capable of specifically binding to a nucleic acid encoding Kir2.3 for diagnosing schizophrenia in a subject by assessing gene expression level of a gene encoding Kir2.3 in peripheral blood lymphocytes taken from said subject.

5. The use of claim 4, wherein the agent is an oligonucleotide specifically hybridizing to a nucleic acid encoding Kir2.3 or a complementer or a part thereof, and preferably the oligonucleotide is a probe having a label, more preferably the label is a fluorescent label or a radioactive label.

6. The use of claim 4, wherein the agent is an oligonucleotide primer useful in PCR.

7. Use of a diagnostic kit for diagnosing schizophrenia in a subject by assessing gene expression level of a gene encoding Kir2.3 in peripheral blood lymphocytes taken from said subject, said kit comprising
at least one agent capable of specifically binding to a nucleic acid encoding Kir2.3 and,
preferably a further agent capable of specifically binding to a nucleic acid encoding DRD2, and
means for producing a detectable signal upon specific binding of the agent.

8. The use of a diagnostic kit according to claim 7 wherein the means for producing a detectable signal comprises a label, preferably an enzymatic label, a fluorescent label or a radioactive label, and
preferably the diagnostic kit further comprises at least one oligonucleotide primer pair useful in amplifying at least one of the nucleic acids.

9. The use of a diagnostic kit according to claim 8 wherein the agent is a probe comprising an oligonucleotide specifically hybridizing to any of the nucleic acids or a complementer or a part thereof, preferably the probe comprises an oligonucleotide and a label bound thereto.

10. The use of a diagnostic kit according to claim 9 wherein the label is a fluorescent label and, preferably,
the probe comprises a fluorescent label and a quencher, and/or
the probe is an oligonucleotide and the label is an intercalating agent, e.g. SYBR-green or ethidium bromide.

11. Use of an oligonucleotide probe for diagnosing schizophrenia in a subject by assessing gene expression level of a gene encoding Kir2.3 in peripheral blood lymphocytes taken from said subject, said probe comprising an oligonucleotide capable of specifically hybridizing to a nucleic acid encoding Kir2.3
or a complementer or a part thereof, wherein
said probe preferably comprises a fluorescent label.

12. A method for diagnosing schizophrenia in a subject comprising
- assessing the gene expression level of at least
a gene encoding DRD2 (dopamine D2 receptor) and, preferably,
a gene encoding Kir2.3 (inwardly rectifying potassium channel 2.3)
in peripheral blood lymphocytes taken from said subject and
- comparing the expression level in the peripheral blood lymphocytes from said subject to a baseline expression level of the respective gene typical of healthy subjects,
wherein a higher gene expression level in the peripheral blood lymphocytes is considered as an indication of the fact that the subject suffers from or is susceptible to schizophrenia.

13. The method of claim 12 wherein assessment of the expression level comprises hybridization of one or more hybridization probe to one or more mRNA obtained from the peripheral blood lymphocytes, said one or more hybridization probe or said one or more mRNA having the sequence
of a gene encoding DRD2 and, preferably,
if gene expression level of a gene encoding Kir2.3 is also assessed, of a gene encoding Kir2.3, or
of a part thereof
or a sequence complementary to any of the above.

14. The method of claim 12 or 13 wherein the expression level is determined by microarray analysis, preferably by a PCR method, preferably a real time PCR method.

15. Use of an agent capable of specifically binding to a nucleic acid encoding DRD2 for diagnosing schizophrenia in a subject by assessing gene expression level of a gene encoding DRD2 in peripheral blood lymphocytes taken from said subject.

16. The use of claim 15, wherein the agent is an oligonucleotide specifically hybridizing to a nucleic acid encoding DRD2, or a complementer or a part thereof, and preferably the oligonucleotide is a probe having a label, more preferably the label is a fluorescent label or a radioactive label.

17. The use of claim 16, wherein the agent is an oligonucleotide primer useful in PCR.

18. Use of a diagnostic kit for diagnosing schizophrenia in a subject by assessing gene expression level of a gene encoding DRD2 in peripheral blood lymphocytes taken from said subject comprising
at least one agent capable of specifically binding to at least a nucleic acid encoding DRD2 and, preferably, a further agent capable of specifically binding to a nucleic acid encoding Kir2.3 and
means for producing a detectable signal upon specific binding of the agent.

19. The use of a diagnostic kit according to claim 18 wherein the means for producing a detectable signal comprises a label, preferably an enzymatic label, a fluorescent label or a radioactive label, and
preferably the diagnostic kit further comprises at least one oligonucleotide primer pair useful in amplifying at least one of the nucleic acids.

20. The use of a diagnostic kit according to claim 18 or 19 wherein the agent is a probe comprising an oligonucleotide specifically hybridizing to any of the nucleic acids or a complementer or a part thereof, preferably the probe comprises an oligonucleotide and a label bound thereto.

21. The use of a diagnostic kit according to claim 20 wherein the label is a fluorescent label and, preferably,
the probe comprises a fluorescent label and a quencher, and/or
the probe is an oligonucleotide and the label is an intercalating agent, e.g. SYBR-green or ethidium bromide.

22. Use of an oligonucleotide probe for diagnosing schizophrenia in a subject by assessing gene expression level of a gene encoding DRD2 in peripheral blood lymphocytes taken from said subject, said probe comprising an oligonucleotide capable of specifically hybridizing to a nucleic acid encoding DRD2
or a complementer or a part thereof, wherein
said probe preferably comprises a fluorescent label.

## Patentansprüche

1. Ein Verfahren zum Diagnostizieren von Schizophrenie bei einem Patienten, umfassend:
- Bemessen des Genexpressionsniveaus von mindestens einem Gen, das für Kir2.3 (einwärts gleichrichtender Kaliumkanal 2.3) kodiert und bevorzugt eines weiteren Gens, das für DRD2 (Dopamin D2-Rezeptor) in peripheren Blutlymphozyten, die von dem Patienten stammen, kodiert; und
- Vergleichen des Expressionsniveaus in den peripheren Blutlymphozyten mit einem Basisexpressionsniveau des entsprechenden Gens, das für gesunde Patienten kennzeichnend ist,
wobei ein höheres Genexpressionsniveau in den peripheren Blutlymphozyten als ein Hinweis dafür gewertet wird, dass der Patient empfänglich für Schizophrenie ist oder daran leidet.

2. Das Verfahren nach Anspruch 1, wobei das Bemessen des Expressionsniveaus das Hybridisieren einer oder mehrerer Hybridisierungssonden mit einer oder mehreren mRNA, die aus den peripheren Blutlymphozyten erhalten wurden, umfasst, wobei die eine oder mehreren Hybridisierungssonden oder die eine oder mehreren mRNAs eine Sequenz
eines Gens aufweisen, das für Kir2.3 kodiert, und bevorzugt,
wenn das Genexpressionsniveau eines Gens, das für DRD2 kodiert, ebenfalls bemessen wird, eines Gens, das für DRD2 kodiert, oder
einen Teil davon
oder eine Sequenz, die zu einer der obigen komplementär ist.

3. Das Verfahren nach Anspruch 1 oder Anspruch 2, wobei das Expressionsniveau mittels Mikroarrayanalysen bestimmt wird, bevorzugt mittels eines PCR-Verfahrens, bevorzugt ein Echtzeit-PCR-Verfahren.

4. Verwendung eines Agens, das spezifisch an eine Nukleinsäure, die für Kir2.3 kodiert, binden kann, zum Diagnostizieren von Schizophrenie bei einem Patienten durch Bemessen des Genexpressionsniveaus eines Gens, das in den peripheren Blutlymphozyten, die von dem Patienten stammen, für Kir2.3 kodiert.

5. Die Verwendung nach Anspruch 4, wobei das Agens ein Oligonukleotid ist, das spezifisch mit einer Nukleinsäure hybridisiert, die für Kir2.3 oder ein Komplementär dazu oder einen Teil davon kodiert, und bevorzugt ist das Oligonukleotid eine Sonde mit einem Marker, noch bevorzugter ist der Marker ein Fluoreszenzmarker oder ein radioaktiver Marker.

6. Die Verwendung nach Anspruch 4, wobei das Agens ein Oligonukleotidprimer ist, der in einer PCR verwendbar ist.

7. Verwendung eines diagnostischen Kits zum Diagnostizieren von Schizophrenie bei einem Patienten durch Bemessen des Genexpressionsniveaus eines Gens, das für Kir2.3 in peripheren Blutlymphozyten, die von dem Patienten stammen, kodiert, wobei das Kit umfasst:
mindestens ein Agens, das spezifisch an eine Nukleinsäure binden kann, die für Kir2.3 kodiert, und
bevorzugt ein weiteres Agens, das spezifisch an eine Nukleinsäure binden kann, die für DRD2 kodiert, und
Mittel zum Erzeugen eines detektierbaren Signals nach dem spezifischem Binden des Agens.

8. Die Verwendung eines diagnostischen Kits nach Anspruch 7,
wobei das Mittel zum Erzeugen eines detektierbaren Signals einen Marker umfasst, bevorzugt einen enzymatischen Marker, eine Fluoreszenzmarker oder einen radioaktiven Marker, und
bevorzugt umfasst das diagnostische Kit weiterhin mindestens ein Oligonukleotid-Primerpaar, das zum Amplifizieren mindestens einer der Nukleinsäuren verwendbar ist.

9. Die Verwendung eines diagnostischen Kits nach Anspruch 8,
wobei das Agens eine Sonde ist, die ein Oligonukleotid umfasst, die mit einer der Nukleinsäuren oder einem Komplementär oder einem Teil davon spezifisch hybridisiert; bevorzugt umfasst die Sonde ein Oligonukleotid und einen daran gebundenen Marker.

10. Die Verwendung eines diagnostischen Kits nach Anspruch 9, wobei der Marker ein Fluoreszenzmarker ist, und, bevorzugt,
umfasst die Sonde einen Fluoreszenzmarker und einen Quencher, und/oder
ist die Sonde ein Oligonukleotid und der Marker ist ein interkalierendes Agens, z.B. SYBR-grün oder Ethidiumbromid.

11. Verwendung einer oligonukleotidsonde zum Diagnostizieren von Schizophrenie bei einem Patienten durch Bemessen des Genexpressionsniveaus eines Gens, das für Kir2.3 in peripheren Blutlymphozyten, die von dem Patienten stammen, kodiert, wobei die Sonde ein Oligonukleotid umfasst, das mit einer Nukleinsäure, die für Kir2.3 kodiert, spezifisch hybridisieren kann,
oder ein Komplementär oder einem Teil davon, wobei
die Sonde bevorzugt einen Fluoreszenzmarker umfasst.

12. Ein Verfahren zum Diagnostizieren von Schizophrenie bei einem Patienten, das umfasst
- Bemessen des Genexpressionsniveaus von mindestens
einem Gen, das für DRD2 (Dopamin D2-Rezeptor) kodiert, und bevorzugt,
einem Gen, das für Kir2.3 (einwärts gerichteter Kaliumkanal 2.3)
in peripheren Blutlymphozyten, die dem Patienten entnommen wurden, kodiert, und
- Vergleichen des Expressionsniveaus in den peripheren Blutlymphozyten des Patienten mit einem Basisexpressionsniveau des entsprechenden Gens, das für gesunde Patienten kennzeichnend ist,
wobei ein höheres Genexpressionsniveau in den peripheren Blutlymphozyten als ein Hinweis dafür gewertet wird, dass der Patient empfänglich für Schizophrenie ist oder daran leidet.

13. Das Verfahren nach Anspruch 12, wobei das Bemessen des Expressionsniveaus das Hybridisieren einer oder mehrerer Hybridisierungssonden an eine oder mehrere mRNAs, die aus peripheren Blutlymphozyten erhalten wurden, umfasst, wobei die eine oder mehreren Hybridisierungssonden oder die eine oder mehreren mRNAs die Sequenz
eines Gens, das für DRD2 kodiert und, bevorzugt,
wenn das Genexpressionsniveaus eines Gens, das für Kir2.3 kodiert, ebenfalls bemessen wird, eines Gens, das für Kir2.3, oder
einen Teil davon
oder eine Sequenz, die zu einer der obigen kompelmentär ist, kodiert, aufweiset.

14. Das Verfahren nach Anspruch 12 oder 13, wobei das Expressionsniveau mittels Mikroarrayanalysen bestimmt wird, bevorzugt mittels eines PCR-Verfahrens, bevorzugt ein Echtzeit-PCR-Verfahren.

15. Verwendung eines Agens, das spezifisch an eine Nukleinsäure, die für DRD2 kodiert, binden kann, zum Diagnostizieren von Schizophrenie bei einem Patienten durch Bemessen des Genexpressionsniveaus eines Gens, das in den peripheren Blutlymphozyten, die von dem Patienten stammen, für DRD2 kodiert.

16. Die Verwendung nach Anspruch 15, wobei das Agens ein Oligonukleotid ist, das spezifisch mit einer Nukleinsäure hybridisiert, die für DRD2 oder ein Komplementär dazu oder einen Teil davon kodiert, und bevorzugt ist das Oligonukleotid eine Sonde mit einem Marker, noch bevorzugter ist der Marker ein Fluoreszenzmarker oder ein radioaktiver Marker.

17. Die Verwendung nach Anspruch 16, wobei das Agens ein Oligonukleotidprimer ist, der in einer PCR verwendbar ist.

18. Verwendung eines diagnostischen Kits zum Diagnostizieren von Schizophrenie bei einem Patienten durch Bemessen des Genexpressionsniveaus eines Gens, das für DRD2 in peripheren Blutlymphozyten, die von dem Patienten stammen, kodiert, wobei das Kit umfasst:
mindestens ein Agens, das spezifisch an mindestens eine Nukleinsäure binden kann, die für DRD2 kodiert, und
bevorzugt ein weiteres Agens, das spezifisch an eine Nukleinsäure binden kann, die für Kir2.3 kodiert, und
Mittel zum Erzeugen eines detektierbaren Signals nach dem spezifischem Binden des Agens.

19. Die Verwendung eines diagnostischen Kits nach Anspruch 18, wobei das Mittel zum Erzeugen eines detektierbaren Signals einen Marker umfasst, bevorzugt einen enzymatischen Marker, eine Fluoreszenzmarker oder einen radioaktiven Marker, und
bevorzugt umfasst das diagnostische Kit weiterhin mindestens ein Oligonukleotid-Primerpaar, das zum Amplifizieren mindestens einer der Nukleinsäuren verwendbar ist.

20. Die Verwendung eines diagnostischen Kits nach Anspruch 18 oder 19, wobei das Agens eine Sonde ist, die ein Oligonukleotid umfasst, die mit einer der Nukleinsäuren oder einem Komplementär oder einem Teil davon spezifisch hybridisiert; bevorzugt umfasst die Sonde ein Oligonukleotid und einen daran gebundenen Marker.

21. Die Verwendung eines diagnostischen Kits nach Anspruch 20, wobei der Marker ein Fluoreszenzmarker ist, und, bevorzugt,
umfasst die Sonde einen Fluoreszenzmarker und einen Quencher, und/oder
ist die Sonde ein Oligonukleotid und der Marker ist ein interkalierendes Agens, z.B. SYBR-grün oder Ethidiumbromid.

22. Verwendung einer Oligonukleotidsonde zum Diagnostizieren von Schizophrenie bei einem Patienten durch Bemessen des Genexpressionsniveaus eines Gens, das für DRD2 in peripheren Blutlymphozyten, die von dem Patienten stammen, kodiert, wobei die Sonde ein Oligonukleotid umfasst, das mit einer Nukleinsäure, die für DRD2 kodiert, spezifisch hybridisieren kann,
oder ein Komplementär oder einem Teil davon, wobei
die Sonde bevorzugt einen Fluoreszenzmarker umfasst.

## Revendications

1. Procédé pour diagnostiquer la schizophrénie chez un sujet comprenant les étapes consistant à :
- évaluer le niveau d'expression génétique d'au moins un gène codant pour le Kir2.3 (le canal potassique 2.3 à rectification intérieure) et, de préférence, un autre gène codant pour le DRD2 (récepteur de la dopamine D2) dans les lymphocytes du sang périphérique recueillis chez ledit sujet et
- comparer le niveau d'expression dans les lymphocytes du sang périphérique à un niveau d'expression initial de base du gène respectif typique de sujets en bonne santé,
dans lequel un niveau d'expression génétique plus élevé dans les lymphocytes du sang périphérique est considéré comme une indication du fait que le sujet souffre de schizophrénie ou est prédisposé à la schizophrénie.

2. Procédé selon la revendication 1, dans lequel l'évaluation du niveau d'expression comprend l'hybridation d'une ou plusieurs sondes d'hybridation à un ou plusieurs ARNm obtenus à partir des lymphocytes du sang périphérique, ladite ou lesdites une ou plusieurs sondes d'hybridation ou ledit ou lesdits un ou plusieurs ARNm ayant une séquence
d'un gène codant pour le Kir2.3 et, de préférence,
si le niveau d'expression génétique d'un gène codant pour le DRD2 est également évalué, d'un gène codant pour le DRD2 ou
d'une partie de celui-ci
ou une séquence complémentaire à l'une quelconque des précédentes.

3. Procédé selon la revendication 1 ou la revendication 2, dans lequel le niveau d'expression est déterminé par analyse sur puce à ADN, de préférence par un procédé d'amplification en chaîne par polymérase (PCR), de préférence par un procédé PCR en temps réel.

4. Utilisation d'un agent capable de se lier spécifiquement à un acide nucléique codant pour le Kir2.3 afin de diagnostiquer la schizophrénie chez un sujet en évaluant le niveau d'expression génétique d'un gène codant pour le Kir2.3 dans les lymphocytes du sang périphérique recueillis chez ledit sujet.

5. Utilisation selon la revendication 4, dans laquelle l'agent est un oligonucléotide s'hybridant spécifiquement à un acide nucléique codant pour le Kir2.3 ou un complément ou une partie de celui-ci, et, de préférence, l'oligonucléotide est une sonde ayant un traceur, de manière davantage préférée le traceur est un traceur fluorescent ou un traceur radioactif.

6. Utilisation selon la revendication 4, dans laquelle l'agent est une amorce d'oligonucléotide utile en PCR.

7. Utilisation d'une trousse de diagnostic pour diagnostiquer la schizophrénie chez un sujet en évaluant le niveau d'expression génétique d'un gène codant pour le Kir2.3 dans les lymphocytes du sang périphérique recueillis chez ledit sujet, ladite trousse comprenant
au moins un agent capable de se lier spécifiquement à un acide nucléique codant pour le Kir2.3 et
de préférence, un autre agent capable de se lier spécifiquement à un acide nucléique codant pour le DRD2, et
un moyen pour produire un signal détectable lors de la liaison spécifique de l'agent.

8. Utilisation d'une trousse de diagnostic selon la revendication 7, dans laquelle le moyen pour produire un signal détectable comprend un traceur, de préférence un traceur enzymatique, un traceur fluorescent ou un traceur radioactif, et
de préférence, la trousse de diagnostic comprend en outre au moins une paire d'amorces d'oligonucléotides utile pour l'amplification d'au moins un des acides nucléiques.

9. Utilisation d'une trousse de diagnostic selon la revendication 8, dans laquelle l'agent est une sonde comprenant un oligonucléotide s'hybridant spécifiquement à l'un quelconque des acides nucléiques, ou un complément ou une partie de celui-ci, la sonde comprend de préférence un oligonucléotide et un traceur lié à celui-ci.

10. Utilisation d'une trousse de diagnostic selon la revendication 9 dans laquelle le traceur est un traceur fluorescent et, de préférence,
la sonde comprend un traceur fluorescent et un désactiveur, et/ou
la sonde est un oligonucléotide et le traceur est un agent intercalant, par exemple le SYBR-green ou le bromure d'éthidium.

11. Utilisation d'une sonde oligonucléotide pour diagnostiquer la schizophrénie chez un sujet en évaluant le niveau d'expression génétique d'un gène codant pour le Kir2.3 dans les lymphocytes du sang périphérique recueillis chez ledit sujet, ladite sonde comprenant un oligonucléotide capable de s'hybrider spécifiquement à un acide nucléique codant pour le Kir2.3
ou un complément ou une partie de celui-ci, dans laquelle
ladite sonde comprend de préférence un traceur fluorescent.

12. Procédé pour diagnostiquer la schizophrénie chez un sujet comprenant les étapes consistant à :
- évaluer le niveau d'expression génétique d'au moins
un gène codant pour le DRD2 (récepteur de la dopamine D2), et, de préférence,
un gène codant pour le Kir2.3 (le canal potassique 2.3 à rectification intérieure)
dans les lymphocytes du sang périphérique recueillis chez ledit sujet et
- comparer le niveau d'expression dans les lymphocytes du sang périphérique dudit sujet à un niveau d'expression initial de base du gène respectif typique de sujets en bonne santé,
dans lequel un niveau d'expression génétique plus élevé dans les lymphocytes du sang périphérique est considéré comme une indication du fait que le sujet souffre de schizophrénie ou est prédisposé à la schizophrénie.

13. Procédé selon la revendication 12, dans lequel l'évaluation du niveau d'expression comprend l'hybridation d'une ou plusieurs sondes d'hybridation à un ou plusieurs ARNm obtenus à partir des lymphocytes du sang périphérique, ladite ou lesdites une ou plusieurs sondes d'hybridation ou ledit ou lesdits un ou plusieurs ARNm ayant la séquence
d'un gène codant pour le DRD2 et, de préférence,
si le niveau d'expression génétique d'un gène codant pour le Kir2.3 est également évalué, d'un gène codant pour le Kir2.3, ou
d'une partie de celui-ci
ou une séquence complémentaire à l'une quelconque des précédentes.

14. Procédé selon la revendication 12 ou 13, dans lequel le niveau d'expression est déterminé par analyse sur puce à ADN, de préférence par un procédé d'amplification en chaîne, par polymérase (PCR), de préférence par un procédé PCR en temps réel.

15. Utilisation d'un agent capable de se lier spécifiquement à un acide nucléique codant pour le DRD2 pour diagnostiquer la schizophrénie chez un sujet en évaluant le niveau d'expression génétique d'un gène codant pour le DRD2 dans les lymphocytes du sang périphérique recueillis chez ledit sujet.

16. Utilisation selon la revendication 15, dans laquelle l'agent est un oligonucléotide s'hybridant spécifiquement à un acide nucléique codant pour le DRD2, ou un complément ou une partie de celui-ci, et, de préférence, l'oligonucléotide est une sonde ayant un traceur, de manière davantage préférée le traceur est un traceur fluorescent ou un traceur radioactif.

17. Utilisation selon la revendication 16, dans laquelle l'agent est une amorce d'oligonucléotide utile en PCR.

18. Utilisation d'une trousse de diagnostic pour diagnostiquer la schizophrénie chez un sujet en évaluant le niveau d'expression génétique d'un gène codant pour le DRD2 dans les lymphocytes du sang périphérique recueillis chez ledit sujet comprenant
au moins un agent capable de se lier spécifiquement à au moins un acide nucléique codant pour le DRD2 et, de préférence, un autre agent capable de se lier spécifiquement à un acide nucléique codant pour le Kir2.3 et
un moyen pour produire un signal détectable lors de la liaison spécifique de l'agent.

19. Utilisation d'une trousse de diagnostic selon la revendication 18, dans laquelle le moyen pour produire un signal détectable comprend un traceur, de préférence un traceur enzymatique, un traceur fluorescent ou un traceur radioactif, et
de préférence, la trousse de diagnostic comprend en outre au moins une paire d'amorces d'oligonucléotides utile dans l'amplification d'au moins un des acides nucléiques.

20. Utilisation d'une trousse de diagnostic selon la revendication 18 ou 19, dans laquelle l'agent est une sonde comprenant un oligonucléotide s'hybridant spécifiquement à l'un quelconque des acides nucléiques, ou un complément ou une partie de celui-ci, la sonde comprend de préférence un oligonucléotide et un traceur lié à celui-ci.

21. Utilisation d'une trousse de diagnostic selon la revendication 20, dans laquelle le traceur est un traceur fluorescent et, de préférence,
la sonde comprend un traceur fluorescent et un désactiveur, et/ou
la sonde est un oligonucléotide et le traceur est un agent intercalant, par exemple le SYBR-green ou le bromure d'éthidium.

22. Utilisation d'une sonde oligonucléotide pour diagnostiquer la schizophrénie chez un sujet en évaluant le niveau d'expression génétique d'un gène codant pour le DRD2 dans les lymphocytes du sang périphérique recueillis chez ledit sujet, ladite sonde comprenant un oligonucléotide capable de s'hybrider spécifiquement à un acide nucléique codant pour le DRD2
ou un complément ou une partie de celui-ci, dans laquelle
ladite sonde comprend de préférence un traceur fluorescent.
